(19) Europäisches Patentamt
European Patent Office
Office européen des brevets

(11) Publication number: **0 128 584**
**B1**

# (12) EUROPEAN PATENT SPECIFICATION

(45) Date of publication of patent specification: **26.10.88**

(51) Int. Cl.$^4$: **C 07 D 307/30,** A 61 K 7/46

(21) Application number: **84106749.9**

(22) Date of filing: **13.06.84**

## (54) Dihydrofuran derivates useful as aroma chemicals, and process for their preparation.

(30) Priority: **13.06.83 US 503952**
**13.06.84 US 503974**

(43) Date of publication of application:
**19.12.84 Bulletin 84/51**

(45) Publication of the grant of the patent:
**26.10.88 Bulletin 88/43**

(84) Designated Contracting States:
**BE CH DE FR GB IT LI NL SE**

(56) References cited:
**US-A-4 198 341**
**US-A-4 252 739**

**J. ORG. CHEM., vol. 45, 1980 M.E. ALONSO et al. "Aluminium Oxide assisted stereo selective rearrangement of Cyclopropyl Retone to 4,5-dihydrofuran" pages 4530-4532**

**CAN. J. CHEM., vol. 50, no. 10, 1973 D.E. MC GREER et al. "A stereochemical and kinetic study of the conversion of methyl cyclopropyl ketones to 4,5-dihydrofurans" pages 1487-1493**

**CHEMICAL ABSTRACTS, vol. 94, no. 3, January 19, 1981, Columbus, Ohio, USA M.G. VINOGRADOV et al. "Selective radical 1,2-addition of carbonyl compounds to conjugated diens" page 415, column 2, abstract-no. 15 458v**

(73) Proprietor: **QUANTUM CHEMICAL CORPORATION**
**99 Park Avenue**
**New York, NY 10016 (US)**

(72) Inventor: **Harris, Eugene G.**
**7019 Sprucehill Circle**
**West Chester Ohio 45069 (US)**
Inventor: **Fayter, Richard G., Jr.**
**2746 Saturn Drive**
**Fairfield Ohio 45014 (US)**
Inventor: **Hall, Allen L.**
**3576 Lewis Road**
**Amelia Ohio 45102 (US)**

(74) Representative: **Patentanwälte Grünecker, Dr. Kinkeldey, Dr. Stockmair, Dr. Schumann, Jakob, Dr. Bezold, Meister, Hilgers, Dr. Meyer-Plath**
**Maximilianstrasse 58**
**D-8000 München 22 (DE)**

(56) References cited:
**CHEMICAL ABSTRACTS, vol. 76, no. 3, January 17, 1972, Columbus, Ohio, USA S. ELKADRI et al. "Isomeric transformation of epoxides with mobile hydrogen atoms in the carbon side chain" page 357, column 2, abstract-no. 14 210z**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**EP 0 128 584 B1**

**Description**

This invention relates to 2,4,5-substituted-2,3-dihydrofurans which are useful fragrance compounds, as well as a process for preparing same.

More specifically, the process involves the catalytic rearrangement (isomerization) of 1,1-disubstituted-2-hydrocarbylcylopropans, wherein one of the substituents in the 1-position is an acyl group, to 2,4,5-substituted-2.3-dihydrofurans.

The use of synthetic fragrance chemicals has added a new dimension to the art of perfumery. As a result of the development of new synthetic perfume chemicals, perfumers have greater flexibility in developing fragrance formulations and are better able to mimic natural aromas. However, as perfumers become more adept in the use of synthetic materials for the formulation of sophisticated fragrances and developing the subtle nuances typically associated with natural fragrances, there is a growing demand for new synthetic fragrance compounds.

Furan derivatives are known to have desireable fragrance and flavour qualities. For example, ethyl furoate, n-amyl furoate, ethyl furylβ-hydroxypropionate and furfural are reported for use in perfumes, cosmetics and soaps. Furfuryl alkyl and aryl ethers are disclosed as flavor enhancers in US—A—3,940,502. Tetrahydrofurans are also disclosed for fragrance applications. US—A—3,668,134 discloses the use of esters or ethers of tetrahydrofurans as perfumery ingredients for detergent compositions. 3-Oximino-4-oxo-2,5-dimethyltetrahydrofuran is described in US—A—4,116,982 and disclosed to have a fine caramel-like fragrance making it useful for the manufacture of scents and flavours. US—A—3,470,209 discloses 2-acetonyl-3,5-dimethyl-5-isopropyl-tetrahydrofuran as having a pleasant spicy odor reminiscent of bay and eucalyptus. 2-(1′-Hydroxymethyl-ethyl)-5-methyl-5-vinyl-tetrahydrofuran is disclosed in US—A—3,764,567 as a useful ingredient for incorporation in floral perfumes. In US—A—3,277,731 carbonates of 1-(α-furyl)-2,2-dialkyl-1,3-dihydroxypropanes and 1-(α-tetrahydrofuryl)-2,2-dialkyl-1,3-dihydroxypropanes are indicated to be useful in perfume compounding.

2,3-Dihydrofurans having a vinyl group at the 2-position, a lower carboxylate radical at the 4-position and a methyl group at the 5-position have been reported in Chemical Abstracts, Volume 75, 87758y (1971); Volume 93; 71095r (1980); Volume 93, 239112v (1980); and Volume 94, 15458v (1981). Additionally, in Chemical Abstracts Volume 96, 52099r (1982), substituted 2,3-dihydrofurans obtained by the oxidative addition of 1,3-dicarbonyl compounds with dienes in the presence of manganese (III) and copper (II) acetates are reported. None of the reported 2,3-dihydrofurans have a higher alkyl group substituted in the 5-position and there is no indication that any of the compounds have useful fragrance qualities.

A procedure for the preparation of substituted dihydrofurans is disclosed in US—A—4,180,446; 4,198,341; 4,198,342 and 4,198,343. The process involves reacting a β-alkoxycrotonic acid ester or 3,3-bisalkoxybutyric acid ester with a 1,1,1-trihalogen-4-methyl-3-pentene-2-ol or 1,1,1-trihalogen-4-methyl-4-pentene-2-ol in the presence of an acid catalyst. 2,4,4-Trimethyl-3-carbalkoxy-5-(β,β-dihalogenvinyl)-4,5-dihydrofurans are produced in the process.

J. Orq. Chem., 45, 4530—4532 (1980) reports the rearrangement of cyclopropyl ketones to 4,5-dihydrofurans at room termperature using aluminium oxide. The rearrangement is accomplished by passing the cyclopropyl ketone in chloroform through a neutral alumina column. Whereas nearly quantitative yields of the dihydrofuran

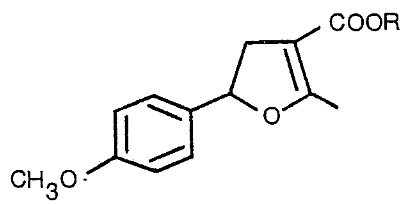

are obtained, contact times of from 24 to 72 hours are required.

Chemical Abstracts, 94, (1981), 15229w describes the ring opening of 1-acetyl-2,2-dichlorocylopropanes in the presence of sodium alcoholates. Depending on the amount of sodium alcoholate used, a dihydrofuran or alkyne is produced.

Dihydrofurans are also reported in Bull. Soc. Chim. France, 1971 (6), 2203-8 as a by-product from the reaction of 1,4-dichlorobutene-2 and sodium ethyl acetylacetate in an alcoholic solution.

US—A—4,252,739 describes a phase-transfer process for the preparation of vinylcyclopropane derivatives by reacting an alkylating agent and an activated methylene compound using an onium catalyst and in the presence of an alkali metal compound and water. Whereas alkylation of the activated carbon atom is predominantly obtained, when the activated methylene compound contains an acyl moiety, e.g. ethyl acetoacetate, up to about 15 per cent dihydrofuran by-product can be obtained as result of alkylation at the oxygen atom of the acyl group.

Dihydrofurans have also been obtained from the rearrangement of acids or light as disclosed in Can. J. Chem., 51 (10), 1487—93 (1973); J. Am. Chem. Soc., 81, 2437—40 (1959); J. Am. Chem. Soc., 69, 30002—3 (1974); and J. Org. Chem., 34, 2301—6 (1969).

While it is known to obtained dihydrofurans from cyclopropyl ketones, it is evident from the above-mentioned references that in most cases a variety of other rearrangement products are produced depending upon the reaction conditions employed.

It is the object of the present invention to provide new dihydrofuran derivatives and a process for preparing same.

The present invention relates to 2,4,5-trisubstituted-2.3-dihydrofurans of the general formula

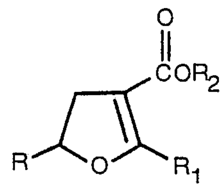

wherein R is an ethyl or vinyl group, $R_2$ is a $C_{1-4}$ alkyl group and $R_1$ is a $C_{3-10}$ hydrocarbon radical.

Said compounds are useful as fragrance compounds for a variety of applications. In the above formula, the hydrocarbon radical $R_1$ can be an aliphatic, cycloaliphatic or aromatic group. When $R_1$ is an aliphatic radical it can be straight-chain or branched and may be saturated or contain unsaturation. Useful cycloaliphatic radicals from which $R_1$ is selected will have from 3 to 6 carbon atoms in the ring and may be saturated or unsaturated. Especially useful cycloaliphatic radicals are the cyclopentyl, cyclohexyl, cyclopentenyl, cyclohexenyl, cyclopentadienyl and cyclohexadienyl which also have one or more alkyl groups substituted thereon. Useful aromatic groups from which $R_1$ is selected include phenyl, benzyl, and alkyl-substituted phenyl or benzyl. $R_2$ can be a straight-chain or branched radical such as methyl, ethyl, n-propyl, iso-propyl, n-butyl, tert-butyl and sec-butyl.

Illustrative compounds within the above definition include:

4-carbomethoxy-5-methyl-2-vinyl-2,3-dihydrofuran;
4-carbomethoxy-5-methyl-2-ethyl-2,3-dihydrofuran;
4-carbethoxy-5-methyl-2-ethyl-2,3-dihydrofuran;
4-carbethoxy-5-methyl-2-vinyl-2,3-dihydrofuran;
4-carbomethoxy-5-ethyl-2-vinyl-2,3-dihydrofuran;
4-carbethoxy-5-ethyl-2-ethyl-2,3-dihydrofuran;
4-carbomethoxy-5-n-pentyl-2-vinyl-2,3-dihydrofuran;
4-carbethoxy-5-n-pentyl-2-vinyl-2,3-dihydrofuran;
4-carbethoxy-5-n-pentyl-2-ethyl-2,3-dihydrofuran;
4-carbethoxy-5-(3-methyl-butyl)-2-vinyl-2,3-dihydrofuran;
4-carbethoxy-5-(3-methyl-butyl)-2-ethyl-2,3-dihydrofuran;
4-carbethoxy-5-(4-methyl-3-pentenyl)-2-vinyl-2,3-dihydrofuran;
4-carbethoxy-5-(4-methyl-3-pentenyl)-2-ethyl-2,3-dihydrofuran;
4-carbomethoxy-5-phenyl-2-vinyl-2,3-dihydrofuran;
4-carbethoxy-5-phenyl-2-vinyl-2,3-dihydrofuran;
4-carbethoxy-5-phenyl-2-ethyl-2,3-dihydrofuran;
4-carbomethoxy-5-benzyl-2-vinyl-2,3-dihydrofuran;
4-carbomethoxy-5-benzyl-2-ethyl-2,3-dihydrofuran;
4-carbethoxy-5-(p-tolyl)-2-ethyl-2,3-dihydrofuran;
4-carboisopropoxy-5-isopropyl-2-vinyl-2,3-dihydrofuran;
4-carboisopropoxy-5-isopropentenyl-2-ethyl-2,3-dihydrofuran.

Particularly useful 2,4,5-trisubstituted-2,3-dihydrofurans, in view of their highly desirable fragrance characteristics, are those compounds where R is ethyl or vinyl, $R_2$ is methyl or ethyl and $R_1$ is a $C_{3-8}$ alkyl or alkenyl group or a cycloaliphatic radical having from 3 to 6 carbon atoms.

The 2,3-dihydrofurans of this invention are generally characterized as having a pleasing, natural fragrance. By varying the ring substituents, it is possible to mimic a number of natural aromas such as, for example, woody, herbaceous, green, nutty, fruity, or vegetable aromas.

Most typically several of these notes will be present in the fragrance even though one note may be predominant. These fragrances are intense, without being overwhelming, and have good diffusivity, stability and dryout characteristics.

The 2,4,5-trisubstituted-2,3-dihydrofurans are useful components in the preparation and formulation of fragranced products such as perfumes, shampoos, deodorants, shaving creams and gels, body lotions and creams, detergent and bar soaps. They must be employed as the sole fragrance material but most generally are used in conjuction with other fragrance materials. When combined with other fragrance materials, the 2,3-dihydrofuran will be present from trace amounts up to about 50 per cent of the fragrance formulation, depending on the particular fragrance desired and the end-use application.

The process of this invention relates to the preparation of said 2,3-dihydrofurans from the corresponding cyclopropyl ketone compound by catalytic isomerization. More specifically, the process involves the rearrangement (isomerization) of 1,1-disubstituted-2-alkylcyclopropanes or 1,1-disubstituted-2-alkenylcyclopropanes, wherein one of the substituents in the 1-position is an acyl group, to 2,4,5-substituted-2,3-dihydrofurans employing an onium compound as the catalyst.

It is also possible with the present proccess to treat products which contain a mixture of the vinylcyclopropane and dihydrofuran moieties to increase the amount of dihydrofuran.

For the process, 1,1-disubstituted-2-hydrocarbylcyclopropanes, wherein one of the substituents in the 1-position is an acyl group, are employed. The 1,1-disubstituted-2-hydrocarbylcyclopropanes, which are also referred to herein as cyclopropyl ketone compounds, correspond to the general formula

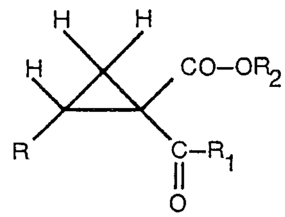

wherein R is an ethyl or a vinyl group $R_1$ is a hydrocarbon radical having from 3 to 10 carbon atoms, and $R_2$ is a $C_{1-4}$ alkyl group. The process further encompasses the use of various geometric and stereo isomers of these cyclopropyl ketones and mixtures and racemates thereof.

For the process of this invention, the cyclopropyl ketone is heated in the presence of an onium catalyst to effect isomerization. The reaction can be represented by the general equation:

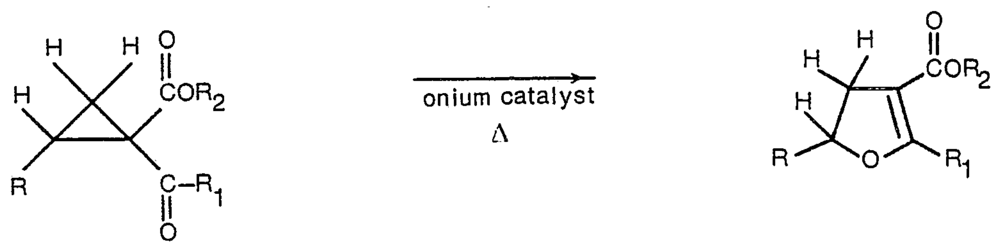


wherein R, $R_1$ and $R_2$ are the same as previously defined.

Temperatures for the reaction range from 60°C to 200°C and best results are obtained at temperatures from 80°C to 170°C. The onium catalyst is employed in an amount from 0.5 to 20 weight per cent, based on the cyclopropyl ketone. Most generally the oniun catalyst is present from 2 to 15 weight per cent.

While it is not necessary for the reaction, the use of a solvent or diluent may be advantageous in some instances. For example, when the cyclopropyl ketone is a solid or viscous fluid, handling of the material can be facilitated by dissolving in a small amount of a suitable solvent or diluent. Any solvent/diluent should be inert and not react with the cyclopropyl ketone, 2,3-dihydrofuran or onium catalyst under the conditions employed for the reaction. It is also advantageous if the solvent has a boiling point above the reaction temperature. Lower boiling solvents can be used, however, this necessitates the use of closed reactors (autoclaves), etc. If the product is to be recovered by distillation, the boiling point of the solvent should also be such that it can be readily separated from the 2,3-dihydrofuran and any unreacted cyclopropyl ketone at the conclusion of the reaction. Useful solvents/diluents which can be used for the process include dimethyl sulfoxide; dimethyl sulfone, sulfolane, dimethyl formamide, N-methyl-2-pyrrolidone, hexamethylphosphoramide; triglyme and the like. In a preferred embodiment of the invention, the onium catalyst is dissolved in the the cyclopropyl ketone and the process is conducted neat, i.e., in the absence of additional solvent or diluent.

Onium compounds useful as catalysts for this invention involve quaternary ammonium and phosphonium compounds having at least 6 carbon atoms and corresponding to the formula

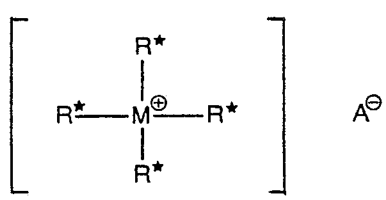

where M is nitrogen or phosphorous, R* represents a hydrocarbon radical having from 1 to 22 carbon atoms and A is a halide, preferably chloride or bromide. The hydrocarbon radicals R* may be the same or different and include alkyl groups, which can be saturated, unsaturated, branched- or straight-chain, phenyl, $C_{1-4}$ alkyl-substituted phenyl, benzyl or $C_{1-4}$ alkyl substituted benzly. Particularly useful onium catalysts contain at least 10 carbon atoms. Illustrative onium compounds which can be used as catalysts for the process of this invention include:

tetrabutylammonium chloride;
tetrabutylammonium bromide;
dimethyldibenzylammonium chloride;
dimethyldibenzylammonium bromide;
trimethylbenzylammonium chloride;
trimethylbenzylammonium bromide;
tricaprylymethylammonium chloride;
tricaprylymethylammonium bromide;
tributylhexadecylphosphonium chloride; and
tributylhexadecylphosphonium bromide.

The cyclopropyl ketones employed in the process should be essetially free of water and caustic. For this reason, cyclopropyl ketones obtained via classical or phase-transfer condensation are generally treated prior to use to remove water, caustic and inorganic salts. For example, in the situation where the cyclopropyl ketone is obtained via the phase-transfer process of US—A—4,252,739, the resulting crude cyclopropyl ketone product may be filtered to remove insoluble inorganic salts or these salts may be dissolved in water followed by separation of the organic and aqueous phases. Residual caustic may be neautralized with dilute acid prior to filtration or dissolution of the salts. The organic phase obtained after the above-treatment may then be treated with drying agents to remove water. Most generally, however, the organic phase is striped or ditstilled under pessure to remove water and any organic solvent present therein.

Example 1

Methyl 1-acetyl-2-vinylcyclopropane-1-carboxylate was prepared by the reaction of 1,4-dichlorobutene-2 and methyl acetoacetate using the classical condensation procesure. For the reaction, methyl sodioacetoacetate was prepared by first reacting 36.6 g sodium metal with 500 ml anhydrous methanol and then adding 185.6 g of the methyl acetoacetic ester. The resulting solution was then added dropwise, at a rate sufficient to maintain reflux, to 100 g. 1,4-dichlorobutene-2 in 200 ml methanol under a nitrogen atmosphere. After addition was complete, refluxing was continued for 3 h followed by stirring overnight at ambient temperature. The resulting reaction product (IA) was then stripped under reduced pressure after filtering to remove insoluble salts. Fractional distillation yielded 55 per cent yield of a product (IB) consisiting predomonantly of the desired vinylcyclopropane product (boiling point 83.5°C—85.0°C at 4.5 mm Hg; $n_D^{22°}$ 1.4705). Nuclear magnetic resonance spectroscopy and gas chromatographic analysis of IB showed the product to consist of 85.7 per cent methyl 1-acetyl-2-vinylcyclopropane-1-carboxylate and 14.3 per cent 4-carbomethoxy-5-methyl-2-vinyl-2,3-dihydrofuran.

To demonstrate the rearrangement, 2 g of IB was combined with 0.24 g tricaprylylmethylammonium chloride and heated at 100°C. After 20 h, the methyl 1-acetyl-2-vinylcyclopropane-1-carboxylate was reduced to 11.6 per cent. The bulk of product (76,3 per cent) was confirmed to be 4-carbomethoxy-5-methyl-2-vinyl-2,3-dihydrofuran. Heating for 20 additional hours increased the amount of 4-carbomethoxy-5-methyl-2-vinyl-2,3-dihydrofuran in the mixture to 83.3 per cent.

When IB was heated for 20 h by itself there was essentially no change in the amounts of 1-acetyl-2-vinylcyclopropane-1-carboxylate and 4-carbomethoxy-5-methyl-2-vinyl-2,3-dihydrofuran — 85.4 per cent and 14.6 per cent, respectively. Heating IA for 20 h at 100°C in a closed reactor (autoslave) gave essentially no change in the relative proportions of the methyl 1-acetyl-2-vinylcyclopropane-1-carboxylate and 4-carbomethoxy-5-methyl-2-vinyl-2,3-dihydrofuran.

Example 2

2 g of product IB was combined with 0.16 g dibenzyldimethylammonium chloride and the mixture heated at 100°C. Nuclear magnetic resonance spectroscopy and gas chromatographic analysis confirmed that the resulting rearranged product, obtained after 20 h, contained 74.5 per cent 4-carbomethoxy-5-methyl-2-vinyl-2,3-dihydrofuran and 25.2 per cent methyl 1-acetyl-2-vinylcyclopropane-1-carboxylate.

Example 3

Product IB (2 g) was combined with 0.30 g tri-n-butylhexadecylphosphonium bromide and the mixture heated at 100°C. After 7 h the amount of methyl 1-acetyl-2-vinylcyclopropane-1-carboxylate present in the mixture was reduced to 52 per cent and 44 per cent 4-carbomethoxy-5-methyl-2-vinyl-2,3-dihydrofuran was present. Continuing the heating for an additional 12 h increased the amount of 4-carbomethoxy-5-methyl-2-vinyl-2,3-dihydrofuran in the mixture to about 75 per cent.

Example 4

To demonstrate the criticality of the catalyst for the rearrangement, Example 3 was repeated except that tetramethylammonium bromide was substituted for the tri-n-butylhexadecylphosphonium bromide at the same mole per cent level. After heating for 20 h at 100°C the amount of 4-carbomethoxy-5-methyl-2-vinyl-2,3-dihydrofuran in the mixture was essentially unchanged.

Example 5

In accordance with the phase transfer procedure of US—A—4,252,739, ethyl 2-vinyl-1-hexanoylocyclopropane-1-caraboxylate was obtained from the reaction of 0.53 mole ethyl hexanoyl acetate

$$(CH_3(CH_2)_4\overset{\overset{O}{\|}}{C}CH_2\overset{\overset{O}{\|}}{C}OC_2H),$$

0.66 mole 1,4-dichlorobutene-2, and 1.06 moles potassium hydroxide. The reaction was carried out in a mixture of water and methylene chloride utilizising 0.0133 mole tricaprylylmethyl-ammonium chloride as the phase transfer catalyst. The resulting reaction product was then filtered through a sintered glass funnel to remove excess potassium hydroxide and insoluble salts formed during the reaction and neutralized with 10 per cent sulfuric acid. The crude product obtained after drying and removal of the methylene chloride contained approximately 95 per cent ethyl 2-vinyl-1-hexanoyl-cyclopropane-1-carboxylate.

The ethyl 2-vinyl-1-hexanoylcyclopropane-1-carboxylate obtained above (120 g) was combined with 25.6 g tricaprylylmethylammonium chloride and heated at 110°C under a nitorogen atmosphere with agitation to isomerize the cyclopropyl ketone to the corresponding 2,3-dihydrofuran. After three hours, gas chromatographic analysis showed the reaction mixture to contained 41 per cent 4-carbethoxy-5-n-pentyl-2-vinyl-2,3-dihydrofuran. Heating was contained at 110°C until the 4-carbethoxy-5-n-pentyl-2-vinyl-2,3-dihydrofuran content was increased to 87 per cent. By distillation it was possible to obtain essentially pure 4-carbethoxy-5-n-pentyl-2-vinyl-2,3-dihydrofuran. The 4-carbethoxy-5-n-pentyl-2-vinyl-2,3-dihydrofuran was a clear colorless liquid (B.P. 103—109°C @ 2 mm Hg; $n_D^{25°}$ 1.4709) and had a pleasing fragrance useful in Jasmone and/or citrus formulations.

nmr($CDCl_3$)σ3 1.28(t, 3H($CH_3$-$CH_2$-O-CO)); 4,23 (g, 2H($CH_3$—$CH_2$—O—CO—)); 0.90 (t,3H($CH_3CH_3$—$CH_2$—$CH_2$—)); 1.10—1.80 (m, 6 methylene H); 2.50—3.55 (m. 2 ring methylene H); 2.70 (br.t., (2 methylene H. adj. to ring)); 4.80—5.55 (m, 3 vinyl H), 5.70—6.34 (m, 1 ring H).

IR (film) 2960, 2930, 2870, 1695, 1637, 1370, 1252, 1225, 1173, 1104, 1050, 970 and 767 $cm^{-1}$.

Example 6

4-Carbethoxy-5-n-pentyl-2-vinyl-2,3-dihydrofuran prepared in accordance with the procedure of Example 1 was hydrogenated to obtain 4-carbethoxy-5-n-pentyl-2-vinyl-2,3-dihydrofuran. For the reaction, 13 g 4-carbethoxy-5-n-pentyl-2-vinyl-2,3-dihydrofuran and 75 ml ethyl acetate were charged to the reactor of a Parr apparatus. The system was thoroughly purged with nitrogen and 0.65 gms of a hydrogenation catalyst (5 per cent palladium on carbon) added under a nitrogen atmosphere. After additional nitrogen purging, shaking was begun and the system pressurized with hydrogen to 103 kPa (15 psig). The system was repressurized as neccesary until no further hydrogen uptake was noted. The reaction mixture was then filtered through Dicalite ® to remove the Pd/carbon catalyst and ethyl acetate removed under reduced pressure. Gas chromatographic anaylysis indicted 98 per cent yield crude 4-carbethoxy-5-n-pentyl-2-ethyl-2,3-dihydrofuran. The crude product was fractionated using spinning-band distillation apparatus to obtain essentially pure 4-carbethoxy-5-n-pentyl-2-vinyl-2,3-dihydrofuran (B.P. 112—114°C @ 1mm Hg; $n_D^{25°}$ 1.4636); which was confirmed by nuclear magnetic reasonance and infrared spectroscopic analysis.

nmr($CDCl_3$)σ 1.29(t, 3H($CH_3$—$CH_2$—O—CO)); 4,19 (q, 2H($CH_3$—$CH_2$—O—CO)); 0.60—1.95 (m 14H(—$CH_2$—$CH_2$ and $CH_3$-$CH_2$-type hydrogens)); 2.30—3.42(m, 2 ring methylene H); 2.68 br.t., (2 methylene H. adj. to ring)); 4.30—4.95 (m, 1 ring H).

IR (film) 2970, 2930, 2870, 1694, 1636, 1462, 1370, 1330, 1255, 1228, 1173, 1104,, 1048, 973 and 765 $cm^{-1}$

The 4-carbethoxy-5-n-pentyl-2-vinyl-2,3-dihydrofuran had notes compatible with and suitable for blending in Jasmone and citrus compositions.

Example 7

In a manner similar to that described in Example 1, 4-carbethoxy-5-(3-methyl-butyl)-2-vinyl-2,3-dihydrofuran was prepared by the isomertization of ethyl 2-vinyl-1-(4-methyl-pentanoyl)cyclopropane-1-carboxylate. For the reaction, 125 ml of the ethyl 2-vinyl-1-(4-methyl-pentanoyl)cyclopropane-1-carboxylate was combined with 25.4 g tricaprylylmethylammonium chloride. The mixture was stirred at 100°C for approximately 11 h, after which time gas chromatographic analysis showed the reaction mixture to contain about 85 per cent 4-carbethoxy-5-(3-methyl-butyl)-2-vinyl-2,3-dihydrofuran. Heating was terminated and the crude product distilled using a spinning-band distillation apparatus fitted with a 30.5 cm (12 inch) column. Essentially pure 4-carbethoxy-5-(3-methyl-butyl)-2-vinyl-2,3-dihydrofuran was obtained. (B.P. 101—112°C @ 1mm Hg; $n_D^{25°}$ 1.4742). The 4-carbethoxy-5-(3-methyl-butyl)-2-vinyl-2,3-dihydrofuran had a pleasing fragrance with a slightly weedy, fatty, nutty-scotch whiskey odor profile.

nmr($CDCl_3$)σ 1.27(t, 3H($CH_3$—$CH_2$—O—CO)); 4,23 (g, 2H($CH_3$—$CH_2$—O—CO—)); 0.90 (d 6H(($CH_3$($_2$—CH)); 1.22—1.82 (m, 3H(—$CH_2$—$CH_2$—CH—)); 2.68( br.t., 2H(2 methylene H. adj. to ring)); 2.40—3.40(m, 2 ring methylene H); 4.85-5.60(m, 3 vinyl H); 5.78—6.40(m, 1 ring H).

IR (film) 2959, 2930, 2875, 1699, 1636, 1369, 1311, 1250, 1234, 1192, 1172, 1135, 1107, 1054, 970 and 765 $cm^{-1}$

Example 8

4-Carbethoxy-5-(3-methyl-butyl)-2-vinyl-2,3-dihydrofuran prepared in accordance with Example 3 was hydrogenated to obtain 4-carbethoxy-5-(3-methyl-butyl)-2-vinyl-2,3-dihydrofuran. The hydrogenation procedure employed was the same as described in Example 2. After removal of the catalyst and ethyl acetate, 17,47 g crude 4-carbethoxy-5-(3-methyl-butyl)-2-vinyl-2,3-dihydrofuran was obtained. Distillation of the crude hydrogenated material yielded essentially pure 4-carbethoxy-5-(3-methyl-butyl)-2-vinyl-2,3-dihydrofuran boiling at 102°C—103°C at 0.6 mm Hg; $n_D^{25°}$ 1.4621). The material had an intense fruity odor-strawberry and apple predominating, with traces of chamomile.

nmr($CDCl_3$)σ 1.28(t, 3H($CH_3$—$CH_2$—O—CO)); 4,21 (g, 2H($CH_3$—$CH_2$—O—CO)); 0.93 (d, 6H($(CH_3)_2$—CH)), 0.71—1.86(m(complex), 8H, 2.68 (br.t, 2H(2 methylene H. adj. to ring)); 2.26—3.31 (m, 2 ring methylene H); 4.28—5.01 (m, 1 ring H)

IR (film) 2980, 2955, 2900, 1705, 1640, 1472, 1375, 1340, 1265, 1240, 1175, 1145, 1110, 1055, 970 and 770 $cm^{-1}$.

Example 9

In a manner similar to that previously described ethyl 2-vinyl-1-(5-methyl-4-hexenoyl)cyclopropane-1-carboxylate, obtained by the condensation of ethyl 5-methyl-4-hexenoylacetate and 1,4-dichlorobutene-2 under phase transfer conditions, was isomerized to obtain 4-carbethoxy-5-(4-methyl-3-pentenyl)-2-vinyl-2,3-dihydrofuran. The isomerization was carried out at 110°C under a nitrogen atmosphere using 19.6 weight per cent tricaprylylmethylammonium chloride catalyst. After about 3 h, chromatographic analysis confirmed the presence of 92 per cent 4-carbethoxy-5-(4-methyl-3-pentenyl)-2-vinyl-2,3-dihydrofuran in the reaction mixture. Distillation of the isomerized product afforded 15 g pure 4-carbethoxy-5-(4-methyl-3-pentenyl)-2-vinyl-2,3-dihydrofuran (B.P. 104—110°C @ 0.6 mm Hg; $n_D^{25°}$ 1.4910).

The clear, colorless liquid had a somewhat hedge-like aroma with a chamomile-like nuance.

nmr($CDCl_3$)σ 1.30(t, 3H($CH_3$—$CH_2$—O—CO)); 4,25 (g, 2H($CH_3$—$CH_2$—O—CO)); 1.68 (d 6H(—$CH_3$($_2$=CH-)), 2.42(br.t, 2H(-$CH_2$-CH=C$(CH_3)_2$)); 2.75 (br.t., 2H(2 methylene H. adj. to ring)); 2.40—3.45 (m, ring methylene H, part. hidden); 4.85—5.60(m, 4 vinyl H); 5.77—6.37(m, 1 ring H).

IR (film) 2975, 2920, 2875, 1691, 1633, 1447, 1372, 1310, 1232, 1155, 1107, 1065, 1025, 972, 915 and 765 $cm^{-1}$

Example 10

4-Carbethoxy-5-(4-methyl-3-pentenyl)-2-vinyl-2,3-dihydrofuran of Example 5 was hydrogenated to obtain 4-carbethoxy-5-(4-methyl-3-pentenyl)-2-ethyl-2,3-dihydrofuran. 5 per cent by weight catalyst (5% Pd on a carbon support) was used. The hydrogenation was essentially complete in 3 h. After removal of the catalyst and ethyl acetate solvent, the crude hydrogenated product was distilled using a 50-plate spinning-band distillation apparatus to provide essentially pure 4-carbethoxy-5-(4-methyl-3-pentenyl)-2-ethyl-2,3-dihydrofuran (B.P. 58°C @ 0.01 mm Hg; $n_D^{25°}$ 1.4791). The product had a nutty character and was smoother in overall odor quality than the unsaturated parent disclosed in Example 5.

nmr($CDCl_3$)σ 1.28(t, 3H($CH_3$—$CH_2$—O—CO)); 4,22 (g, 2H($CH_3$—$CH_2$—O—CO)); 0.98(t 3H($CH_3$—$CH_2$—C—)); 1.33—1.70)q, 2H($CH_3$—$CH_2$—C—)hidden); 1.68(d, 6H(($CH_{32}$C=CH—)); 2.35(br.t., 2H(-$CH_2$—CH=C($CH_3$($_2$)); 2.73(br.t., 2H(2 methylene H adj. to ring)); 2.30—3.35(m, 2 ring methylene H, part. hidden); 4.30—4.80(m, 1 ring H); 5.23(br.t., 1 vinyl H).

IR (film) 2970, 2930, 2875, 1690, 1635, 1450, 1373, 1236, 1156, 1107, 1066, 1026, 985, 830 and 765 $cm^{-1}$

Example 11

4-Carbethoxy-5-phenyl-2-vinyl-2,3-dihydrofuran was obtained in the usual manner by isomerizing ethyl 2-vinyl-1-benzoylcyclopropane-1-carboxylate. To catalyze the isomerization, tricaprylylmethylamonium chloride was employed at a 20 per cent weight level. The mixture was heated at 150°C for about two hours after which time only about 7 per cent of the cyclopropyl ketone remained. After two distillations of the resulting isomerized product, essentially pure 4-carbethoxy-5-ohenyl-2-vinyl-2,3-dihydrofuran was obtained (B.P. 110°C @ 0.1 mm Hg; $n_D^{25°}$ 1.5570). The product has a pleasing aroma that was somewhat coumarin-like in odor character.

nmr($CDCl_3$)σ 1.20(t, 3H($CH_3$—$CH_2$—O—CO)); 4,17 (q, 2H($CH_3$—$CH_2$—O—CO)); 2.70—3.60(oct, 2 ring methylene H); 2.93—5.60(m, 3 vinyl H); 5.80—6.45(m, 1 ring H); 7.30—8.15(m, 5 phenyl H).

IR (film) 2980, 1697, 1622, 1598, 1496, 1446, 1370, 1240, 1085, 1070, 987, 926, 760 and 695 $cm^{-1}$

Example 12

Various products obtained from phase-transfer reactions similar to that of Examples 7—11 and containing varying amounts of cyclopropyl ketone were rearranged in accordance with the process of the present invention. All of the products were neutralized and essentially free of insoluble salts, water and inorganic solvents. Tricaprylylmethylammonium chloride was employed as the catalyst for all of the rearrangement reactions. Details of these and some of the previous reactions, including the amount of the cyclopropyl ketone and corresponding 2,3-dihydrofuran, are provided in Table I. By-products present in the starting material or obtained from the rearrangement are not identified.

TABLE 1

| | Starting Material | WT Catalyst | Reaction Temp (°C) | Reaction Time (hours) | | Final Product |
|---|---|---|---|---|---|---|
| 77.8% | ethyl 2-vinyl-1-(4-methylpentanoyl)-cyclopropane-1-carboxylate | 20.3 | 110 | 10 | 17.4% | ethyl 2-vinyl-1-(4-methylpentanoyl)-cyclopropane-1-carboxylate |
| 22.2% | 4-carbethoxy-5-(3-methylbutyl)-2-vinyl-2,3-dihydrofuran | | | | 82.6% | 4-carbethoxy-5-(3-methylbutyl)-2-vinyl-2,3-dihydrofuran |
| 75% | ethyl 2-vinyl-1-(5-methyl-4-hexenoyl)-cyclopropane -1-carboxylate | 19.6 | 110 | 3 | 8% | ethyl 2-vinyl-1-(5-methyl-2-hexenoyl)-cyclopropane -1-carboxylate |
| 25% | 4-carbethoxy-5-(4-methyl-3-pentenyl)-2-vinyl-2.3-dihydrofuran | | | | 92% | 4-carbethoxy-5-(4-methyl-3-pentenyl)-2-vinyl-2.3-dihydrofuran |
| 79% | ethyl 2-vinyl-1-benzoylcyclopropane-1-carboxylate | 20.0 | 150 | 2 | 7% | ethyl 2-vinyl-1-benzoylcyclopropane-1-carboxylate |
| 15% | 4-carbethoxy-5-phenyl-2-vinyl-2,3-dihydrofuran | | | | 87% | 4-carbethoxy-5-phenyl-2-vinyl-2,3-dihydrofuran |

**Claims**

1. A dihydrofuran derivative of the formula

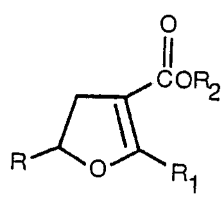

wherein R is an ethyl or vinyl group, $R_1$ is a hydrocarbon radical having from 3 to 10 carbon atoms and $R_2$ is a $C_{1-4}$ alkyl group.

2. The compound of Claim 1 wherein $R_1$ is an aliphatic radical.

3. The compound of Claim 1 or 2 wherein $R_1$ is a $C_{3-8}$ alkyl or alkenyl group or a cycloaliphatic radical having from 3 to 6 carbon atoms and $R_2$ is methyl or ethyl.

4. The compound of Claim 3 wherein $R_2$ is methyl or ethyl and the cycloaliphatic radical is cyclopentyl, cyclohexyl, cyclopentenyl, cyclohexenyl, cyclopentadienyl or cyclohexadienyl.

5. The compound of Claim 1 wherein $R_1$ is an aromtic radical selected from phenyl, benzyl and alkyl-substituted pehnyl or benzyl.

6. The compound of any of Claims 1, 2 or 5 wherein $R_2$ is methyl or ethyl.

7. The compound of Claim 1 which is 4-carbethoxy-5-n-pentyl-2-vinyl-2,3-dihydrofuran, 4-carbethoxy-5-n-pentyl-2-ethyl-2,3-dihydrofuran, 4-carbethoxy-5-(3-methyl-butyl)-2-vinyl-2,3-dihydrofuran, 4-carbethoxy-5-(3-methyl-butyl)-2-ethyl-2,3-dihydrofuran, 4-carbethoxy-5-(4-methyl-3-pentenyl)-2-vinyl-2.3-dihydrofuran.

8. A fragrance composition characterized in that the composition contains a dihydrofuran derivative of any of Claims 1 to 7.

9. A process for prepariang the dihydrofuran derivative of any of claims 1 to 7 which comprises heating the cyclopropyl ketone which is essentially free of water and caustic and corresponds to the formula

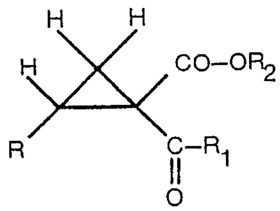

wherein R is an ethyl or a vinyl group, $R_1$ is a hydrocarbon radical having from 3 to 10 carbon atoms and $R_2$ is a $C_{1-4}$ alkyl group, at a temperature in the range 60°C to 200°C and in the presence of 0.5 to 20 weight per cent of an onium catalyst, based on the weight of the cyclopropyl ketone, to effect isomerization.

10. The process of claim 9 wherein the onium catalyst is a quaternary ammonium or quaternary phosphonium compound containing at least 6 carbon atoms and corresponding to the formula

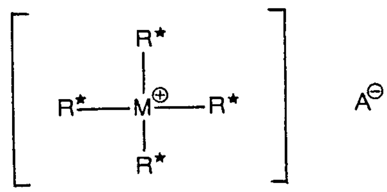

where M is nitorgen or phosphorus, R* represents a hydrocarbon radical having from 1 to 22 carbon atoms and A is a halide.

11. The process of Claim 9 or 10 wherein the temperature is in the range of 80°C to 170°C.

12. The process of Claim 10 or 11 wherein the onium catalyst contains at least 10 carbon atoms with R* being an alkyl group, phenyl, $C_{1-4}$ alkyl-substituted phenyl, benzyl, or $C_{1-4}$ alkyl-substituted benzyl and $A^-$ being chloride or bromide.

13. The process of any of Claims 9 to 12 wherein the onium catalyst is present in an amount from 2 to 15 per cent.

**Patentansprüche**

1. Dihydrofuranderivat der Formel

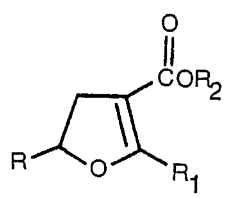

worin R eine Ethyl-oder Vinylgruppe ist, $R_1$ ein Kohlenwasserstoffrest mit 3 bis 10 Kohlenstoffatomen ist und $R_2$ eine $C_{1-4}$-Alkylgruppe ist.

2. Verbindung nach Anspruch 1, worin $R_1$ ein aliphatischer Rest ist.

3. Verbindung nach Anspruch 1 oder 2, worin $R_1$ eine $C_{3-8}$-Alkyl- oder Alkenylgruppe oder ein cycloaliphtischer Rest mit 3 bis 6 Kohlenstoffatomen ist und $R_2$ Methyl oder Ethyl ist.

4. Verbindung nach Anspurch 3, worin $R_2$ Methyl oder Ethyl ist und der cycloaliphatische Rest Cyclopentyl, Cyclohexyl, Cyclopentenyl, Cyclohexenyl, Cyclopentadienyl oder Cyclohexadienyl ist.

5. Verbindung nach Asnpruch 1, worin $R_1$ ein aromataischer Rest, gewählt aus Phenyl, Benzyl und alkylsubstituiertem Phenyl oder Benzyl, ist.

6. Verbindung nach einem der Ansprüche 1, 2 oder 5, worin $R_2$ Methyl oder Ethyl ist.

7. Verbindung nach Anspruch 1, welche 4-Carbethoxy-5-n-pentyl-2-vinyl-2,3-dihydrofuran, 4-Carbethoxy-5-n-pentyl-2-ethyl-2,3-dihydrofuran, 4-Carbethoxy-5-(3-methyl-butyl)-2-vinyl-2,3-dihydrofuran, 4-Carbethoxy-5-(3-methyl-butyl)-2-ethyl-2,3-dihydrofuran, 4-Carbethoxy-5-(4-methyl-3-pentennyl);-2-vinyl-2,3-dihydrofuran ist.

8. Duftzuasammensetzung, dadurch gekennzeichnet, daß die Zuusammensetzung ein Dihydrofuranderivat nach einem der Ansprüche 1 bis 7 enthält.

9. Verfaharen zur Herstellung des Dihydrofuranderivats nach einem der Ansprüche 1 bis 7, bei dem das Cyclopropylketon, das im wesentlichen wasserfrei und kaustisch ist und der Formel

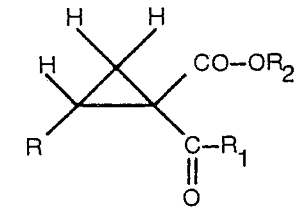

entspricht, worin, R eine Ethyl-oder Vinylgruppe ist, $R_1$ ein Kohlenwasserstoffrest mit 3 bis 10 Kohlenstoffatomen ist und $R_2$ eine $C_{1-4}$ Alkylgruppe ist, bei einer Temperatur im Bereich von 60°C bis 200°C und in Gegenwart von 0,5 bis 20 Gew.-% eines Oniumkatalysators, bezogen auf das Gewicht des Cyclopropylketons, erwärmt wird, um eine Isomerisation zu bewirken.

10. Verfahren nach Anspruch 9, worin der Oniumkatalysator eine quaternäre Ammonium- oder quaternäre Phosphoniumverbindung ist, die wenigstens 6 Kohlenstoffatome enthält und der Formel

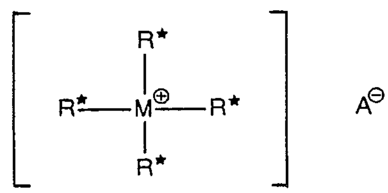

entspricht, worin M Stickstoff oder Phosphor ist, R* einen Kohlenwasserstoffrest mit 1 bis 22 Kohlenstoffatomen bedeutet und A ein Halogenid ist.

11. Verfahren nach Anspruch 9 oder 10, worin die Temperatur im Bereich von 80°C bis 170°C liegt.

12. Verfahren nach Anspruch 10 oder 11, worin der Oniumkatalysator wenigstens 10 Kohlenstoffatome enthält, wobei R* eine Alkylgruppe, Phenyl, $C_{1-4}$-alkylsubstituiertes Phenyl, Benzyl, oder $C_{1-4}$-alkylsubstituiertes Benzyl ist und $A^-$ Chlorid oder Bromid ist.

13 Verfahren nach einem der Anspruche 9 bis 12, worin der Oniumkatalysator in einer Menge von 2 bis 15% worliegt.

**Revendications**

1. Dérivé de dihydrofuranne de formule:

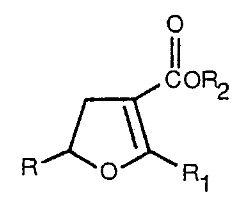

dans laquelle:
—R représente un groupe éthyle ou vinyle;
—$R_1$ représente un radical hydrocarboné ayant de 3 à 10 atomes de carbone; et
—$R_2$ représente un groupe alkyle en $C_{1-4}$.

2. Composé selon la revendication 1, dans lequel $R_1$ représente un radical aliphatique.

3. Composé selon la revendication 1 ou 2, dans lequel:
— $R_1$ représente une groupe alkyle ou alcényle en $C_{3-8}$, ou bien un radical cycloaliphatique ay ant de 3 à 6 atomes de carbone; et
— $R_2$ représente méthyle ou éthyle.

4. Composé selon la revendication 3, dans lequel $R_2$ représente méthyle ou éthyle et le radical cycloaliphatique est cyclopentyle, cyclohexyle, cyclopentényle, cyclohexényle, cyclopentadiényle ou cyclohexadiényle.

5. Composé selon la revendication 1, dans lequel $R_1$ représente un radical aromatique choisi parmi phényle, benzyle et phényle ou benzyle substitués par alkyle.

6. Composé selon l'une des revendications 1, 2 ou 5, dans lequel $R_2$ représente méthyle ou éthyle.

7. Composé selon la revendication 1 qui est:
le carbéthoxy-4 n-pentyl-5 vinyl-2 dihydro-2,3 furanne;
le carbéthoxy-4 n-pentyl-5 éthyl-2 dihydro-2,3 furanne;
le carbéthoxy-4 (méthyl-3 butyl)-5 vinyl-2 dihydro-2,3 furanne;
le carbéthoxy-4 (méthyl-3 butyl)-5 éthyl-2 dihydro-2,3 furanne;

le carbéthoxy-4 (méthyl-4 pentényl-3)-5 vinyl-2 dihydro-2,3 furanne.

8. Compositoin de parfum, caractérisée par le fait que la composition contient un dérivé de dihydrofuranne tel que défini à l'une des revendications 1 à 7.

9. Procédé de préparation de dérivé de dihydrofuranne tel que défini à l'une des revendications 1 à 7, qui consiste à chauffer la cyclopropyl cétone qui est sensiblement exempte d'eau et caustique, et qui correspond à le formule:

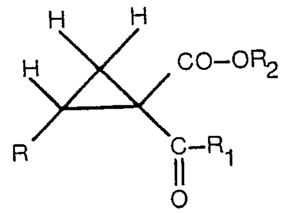

dans laquelle:

— R représente un groupe éthyle ou vinyle;

— $R_1$ représente un radical hydrocarboné ayant de 3 à 10 atomes de carbone; et

— $R_2$ représente un groupe alkyle en $C_{1-4}$, à une température se situant dans la plage de 60°C à 200°C et en pré sence de 0,5 à 20 pour cent en poids d'un catalyseur de type onium, sur la base du poids de la cyclopropyl cétone, pour effectuer une isomérisation.

10. Procédé selon la revendication 9, dans lequel le catalysur de type onium est un composé d'ammonium quaternaire ou de phosphonium quaternaire, contenant au moins 6 atomes de carbone et correspondant à la formule:

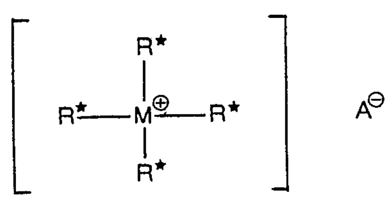

dans laquelle:

—M représente azote ou phosphore;

— R* représente un radical hydrocarboné ayant de 1 à 22 atomes de carbone; et

— A représente un halogénure.

11. Procédé selon la revendication 9 ou 10, dans lequel la température se situe dans la plage de 80°C à 170°C.

12. Procédé selon la revendication 10 ou 11, dans lequel la catalyseur de type onium contient au moins 10 atomes de carbone, R* représentant un groupe alkyle, phényle, phényle substitué par alkyle en $C_{1-4}$, benzyle ou benzyle substitué par alkyle en $C_{1-4}$, et $A^{\ominus}$ représentant chlorure un bromure.

13. Procédé selon l'une des revendications 9 à 12, dans lequel le catalyseur de type onium est présent en une quantité allant de 2 à 15 pour cent.